(19) Europäisches Patentamt
European Patent Office
Office européen des brevets

(11) Publication number: 0 053 025
B1

(12) EUROPEAN PATENT SPECIFICATION

(45) Date of publication of patent specification: 10.04.85

(51) Int. Cl.$^4$: C 07 H 19/24, C 12 P 19/38, A 61 K 31/535

(21) Application number: 81305505.0

(22) Date of filing: 20.11.81

# (54) Antibiotic and its preparation from a streptomyces microorganism.

(30) Priority: 22.11.80 JP 165159/80

(43) Date of publication of application:
02.06.82 Bulletin 82/22

(45) Publication of the grant of the patent:
10.04.85 Bulletin 85/15

(84) Designated Contracting States:
DE FR GB IT

(56) References cited:
EP-A-0 038 568

CHEMICAL ABSTRACTS, vol. 92, no. 17, 28th April 1980, page 628, no. 147110u, Columbus Ohio (USA); A.A. AKHREM et al.: "Nucleosides of 6-methyluracil"

HAMAO UMEZAWA, Index of antibiotics from actinomycetes, volume II, Japan scientific societies press, Tokyo, page 1072, "A-201 A".

CHEMICAL ABSTRACTS, vol. 96, 1982, page 627, no. 85911c, Columbus Ohio (USA); H. NAKAMURA et al.: "The x-ray structure determination of exanosine"

(73) Proprietor: ZAIDAN HOJIN BISEIBUTSU KAGAKU KENKYU KAI
14-23, Kami Osaki 3-chome
Shinagawa-ku Tokyo (JP)

(72) Inventor: Umezawa, Hamao
4-23 Toyotama-kita
Nerima-ku Tokyo (JP)
Inventor: Shimada, Nobuyoshi
2-9-10 Shimo
Kita-ku Tokyo (JP)
Inventor: Naganawa, Hiroshi
3-17 Honmachi Denenchofu
Ota-ku Tokyo (JP)
Inventor: Takita, Tomohisa
7-10-15 Negishidai
Asaka-shi Saitama-ken (JP)
Inventor: Hamada, Masa
1-26 Naito-Machi
Shinjuku-ku Tokyo (JP)
Inventor: Takeuchi, Tomio
5-1-11 Higashigotanda
Shinagawa-ku Tokyo (JP)

(74) Representative: Woods, Geoffrey Corlett et al
J.A. KEMP & CO. 14 South Square Gray's Inn
London WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 053 025 B1

Courier Press, Leamington Spa, England.

(56) References cited:
**CHEMICAL ABSTRACTS, vol. 96, 1982, page 292, no. 48667g, Columbus Ohio (USA); N. SHIMADA et al.: "Oxanosine, a novel nucleoside from actinomycetes".**

**Description**

The present invention is concerned with a new antibiotic.

Chemical Abstracts, volume 92, no. 17, 1980, page 628, 147110u (Akhrem et al, Vesti Akad. Navuk BSSR, Ser. Khim. Navuk 1979, (6), 103—9) describes the preparation of certain nucleosides of 6-methyl-1,3-oxazine-2,4-dione and 6-methyluracil. The Index of Antibiotics from Actinomycetes, volume II, Japan Scientific Societies Press, Tokyo, Editor-in-Chief Hamao Umezawa, A-201 A discloses a nucleoside produced by *Streptomyces capreolus* NRRL 3817 which has different properties from the antibiotic of the present invention.

EP—A—0038568 (Wellcome Foundation Limited) describes and claims a method for the preparation of 4-substituted-1-β-*D*-ribofuranyl-1*H*-imidazo-[4,5-*c*]-pyridines of formula:

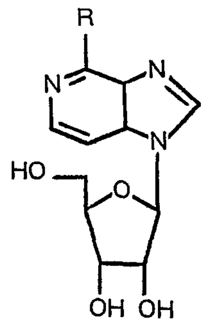

in which R in particular is amino, halogeno, thiol, alkylthio or substituted amino, by reaction of a 4-substituted-1*H*-imidazo-[4,5-*c*]pyridine with a ribose donor system comprising ribose-1-phosphate and a phosphorylase type enzyme. EP—A—0038568 was published after the Japanese priority date but before the EPC filing date of the present case.

The present invention provides an antibiotic referred to herein as Oxanosine and represented by the following formula (I):

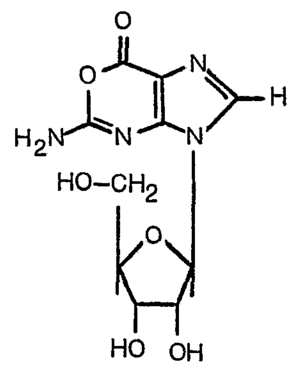

(I)

and acid addition salts thereof.

Oxanosine exerts a growth-inhibiting action on gram-negative bacteria and has anti-HeLa (carcinostatic) and anti-viral actions. Oxanosine is therefore valuable as a medicine. The present invention thus also provides a pharmaceutical composition comprising Oxanosine or an acid addition salt thereof as active ingredient, together with a pharmaceutically acceptable carrier or diluent.

Oxanosine or an acid addition salt thereof is obtained in accordance with the present invention by culturing the Oxanosine-producing microorganism *Streptomyces capreolus* MG265-CF3, collecting the Oxanosine from the culture broth and, if desired, converting said compound to an acid addition salt thereof.

*Streptomyces capreolus* MG265-CF3 was isolated from soil collected at the compound of Institute of Microbial Chemistry in July 1979 and mutants thereof. This strain MG265-CF3 was deposited at Fermentation Research Institute, Agency of Industrial Science and Technology (located at Yatabe-cho, Tsukuba-gun, Ibaragi-ken, Japan) under a deposit number of FERM P No. 5735 on October 6, 1980, and at American Type Culture Collection, Parklawn Drive, Rockville, Maryland, U.S.A., under a deposit number of ATCC 31963 on September 14, 1981.

The mycological properties of the strain MG265-CF3 are described below.

1. Morphology:

Under a microscope, it is seen that strain MG265-CF3 forms relatively long, straight aerial mycelia from branched substrate mycelia. Formation of spirals or loops is not observed. Mature spore chains include at least 10 spores, and the size of spores is (0.4—0.5 microns)×(1.5—2.4 microns). The surfaces of spores are smooth.

2. Cultural characteristics on various culture media:

The colors in parenthesis are described according to "Color Harmony Manual" of Container Corporation of America.

(1) Sucrose nitrate agar (cultured at 27°C):

White to brownish white [3ba, Pearl] aerial mycelia adhere on a colorless, pale yellow or pale yellowish brown [2le, Mustard] growth. A soluble pigment giving a faint yellowish tint is produced.

(2) Glucose-asparagine agar (cultured at 27°C):

White aerial mycelia adhere thinly on a pale yellow to pale yellowish brown [2gc, Bamboo to 3ic, Lt Amber] growth, and a soluble pigment giving a faint yellowish tint is formed.

(3) Glycerol-asparagine agar (International Streptomyces Project (ISP) Medium 5 cultured at 27°C):

White to yellowish gray aerial mycelia adhere thinly on a colorless, pale yellow or pale yellowish brown [2gc, Bamboo to 3ne, Topaz] growth, and a soluble pigment giving a faint yellowish tint is formed.

(4) Inorganic salt-starch agar (ISP Medium 4 cultured at 27°C):

White aerial mycelia adhere on a colorless to pale yellowish brown [2gc, Bamboo to 2pe, Mustard Gold] growth. The medium is tinted brown only very slightly and no substantial formation of a soluble pigment is observed.

(5) Tyrosine agar (ISP Mediums cultured at 27°C):

White to yellowish gray [3ba, Pearl] aerial mycelia adhere on a colorless, pale yellow or pale yellowish brown [2le, Mustard to 3le, Cinnamon] growth. A soluble pigment giving a faint yellowish brown tint is formed.

(6) Nutrient agar (cultured at 27°C):

The growth is colorless to pale yellowish brown [3ne, Topax], and adhesion of aerial mycelia is not observed. Formation of a soluble pigment is not observed.

(7) Yeast extract-malt extract agar (ISP Medium 2 cultured at 27°C):

White aerial mycelia adhere slightly on a colorless to pale yellowish brown [3ne, Topaz] growth, and formation of a soluble pigment is not observed.

(8) Oat meal agar medium (ISP Medium 3 cultured at 27°C):

White aerial mycelia adhere on a pale yellow to pale yellowish brown [2ic, Honey Gold to 2pe, Mustard Gold] growth, and a soluble pigment giving a yellow tint is formed.

(9) Glycerol nitrate agar (cultured at 27°C):

White to yellowish gray [2ba, Pearl] aerial mycelia adhere on a colorless to pale yellowish brown [2gc, Bamboo to 2ne, Mustard Gold] growth, and a soluble pigment giving a faint yellowish tint is formed.

(10) Starch agar (cultured at 27°C):

White to yellowish gray [3ba, Pearl] aerial mycelia adhere thinly on a colorless, pale yellow or dull yellow orange [3ic, Lt Amber to 3ne, Topaz] growth, and formation of a soluble pigment is not observed.

(11) Calcium malate agar (cultured at 27°C):

White to yellowish gray [3ba, Pearl] aerial mycelia adhere on a colorless to pale yellow [2gc, Bamboo] growth. Formation of a soluble pigment is not observed.

(12) Cellulose (cultured at 27°C):

White aerial mycelia adhere thickly on a colorless growth, and a soluble pigment giving a faint yellowish brown tint is formed.

(13) Gelatin stab culture (15% plain gelatin, cultured at 20°C; glucose-peptone gelatin, cultured at 27°C):

In each culture medium, the growth is colorless to pale yellow, and no aerial mycelium adheres and formation of a soluble pigment is not observed.

(14) Skim milk (cultured at 37°C):

The growth is pale yellow, pale yellowish brown or pale yellow orange [3ic, Lt Amber] and no aerial mycelium adheres. A pale yellow orange soluble pigment is formed.

3. Physiological properties:

(1) Growth temperature range:

Culture tests were carried out at 20, 24, 27, 30, 37 and 50°C in a glucose-asparagine agar medium.

Growth was observed at the respective temperatures except 50°C. It is deemed that the optimum temperature is in the range of from 30 to 37°C.

(2) Liquefaction of gelatin (15% plain gelatin, cultured at 20°C; glucose-peptone gelatin, cultured at 27°C):
In the case of a plain gelatin medium, liquefaction starts 10 days after the initiation of culturing, but liquefaction is not completed within 3 weeks. The liquefaction is moderate to weak. In the case of a glucose-peptone gelatin medium, liquefaction of gelatin starts after about 21 days from initiation of culturing. The liquefaction is weak.

(3) Hydrolysis of starch (inorganic salts-starch agar medium and starch agar medium, cultured at 27°C):
Hydrolysis is observed after 5 days from initiation of culturing, and the hydrolysis activity is moderate to strong.

(4) Coagulation and peptonization of skim milk (cultured at 37°C in skim milk):
No change is observed during a period of 8 days from initiation of culturing. Coagulation is completed on the 10th day and peptonization starts on the 10th day and is completed on the 21st day. The activity is moderate.

(5) Formation of melanin pigment (tryptone-yeast extract broth ISP Medium 1, peptone-yeast extract iron agar ISP Medium 6, tyrosine agar ISP Medium 7, cultured at 27°C):
Formation of a melanin pigment is not observed in any of these culture media.

(6) Utilization of carbon sources (Pridham and Gottlieb agar medium, ISP Medium 9, cultured at 27°C):
L-Arabinose, D-xylose, G-glucose and D-fructose are utilized for growing, but L-rhamnose, raffinose and D-mannitol are not utilized. It is judged that inositol is probably utilized, and sucrose is utilized in some cases but not utilized in other cases.

(7) Dissolution of calcium malate (calcium malate agar medium, cultured at 27°C):
Calcium malate is dissolved in the periphery of the growth after 7 days from initiation of culturing, and the dissolving action is moderate to strong.

(8) Reduction of nitrate (aqueous peptone containing 1.0% of potassium nitrate, ISP Medium 8, cultured at 27°C):
Negative.

(9) Decomposition of cellulose (cultured at 27°C):
Negative.

It can be seen from these properties that strain MG265-CF3 belongs to the genus *Streptomyces.* Neither whirl formation nor spirals are observed on aerial mycelia, and the surfaces of spores are smooth. White to yellowish gray aerial mycelia adhere on pale yellow, pale yellowish brown or dull yellow orange growths on various culture media, and soluble pigments giving only a faint yellow to yellowish brown tint are formed.

In each culture medium, formation of a melanin is negative, and the proteolytic action is moderate to weak and the starch hydrolyzing action is moderate to strong.

When known strains having similar properties are considered, it is seen that *Streptomyces capreolus* [Journal of Systematic Bacteriology, *18*, pages 304—305, 1968 (reference 1) and Antimicrobial Agents and Chemotherapy, pages 596—606, 1962 (reference 2)] is closely related to the strain MG265-CF3. *Streptomyces capreolus st.* ISP 5225 is compared with strain MG265-CF3 in Table 1.

Strain MG265-CF3 was isolated from the soil collected from the compound of Institute of Microbial Chemistry as follows:

An amount of soil was collected and after dispersing the soil into a suitable amount of sterilized water, the supernatant layer was suitably diluted. The diluted liquid was sprayed onto a culture medium, for separation, in which the actinomycetes can grow. The culture medium was cultured in an incubator at 27 to 30°C. After confirming the appearance of colonies of actinomycetes, the colonies were separated from the culture medium. From the thus separated colonies, the strain MG265-CF3 was isolated.

TABLE 1

| | MG265-CF3 | *Streptomyces capreolus* ISP 5225 |
|---|---|---|
| Aerial mycelium | rectiflexibiles | rectiflexibiles |
| Spore surface | smooth | smooth |
| Color of aerial mycelium | white to yellowish gray | white to yellowish gray |
| Color of growth | pale yellow, pale yellowish brown or dull yellow orange | pale yellow, pale yellowish brown or dull orange |
| Soluble pigment | yellow to yellowish brown | yellowish brown to brown |
| Formation of melanin | | |
| ISP Medium 1 | − | − |
| ISP Medium 6 | − | − |
| ISP Medium 7 | − | − |
| Hydrolysis of starch | + | + |
| Coagulation of milk | + | − |
| Peptonization of milk | + | − |
| Liquefaction of gelatin | | |
| Plain gelatin | + (moderate to weak) | + (moderate to weak) |
| glucose-peptone gelatin | ± | − |
| Reduction of nitrate | − | − |
| Utilization of carbon sources | | |
| D-glucose | + | + |
| L-arabinose | + | + |
| D-xylose | + | + |
| D-fructose | + | + |
| sucrose | + or − | − |
| inositol | ± | − |
| L-rhamnose | − | − |
| raffinose | − | − |
| D-mannitol | − | + (+ or − according to reference 1) |

Note: "±": probably positive.

As is apparent from the results shown in Table 1, the MG265-CF3 strain is quite in agreement with *Streptomyces capreolus st.* ISP 5225 in coagulation and peptonization of milk, liquefaction of glucose-peptone gelatin and utilization of sucrose, inositol and D-mannitol except for minor differences. All the cell

components of both the strains were determined according to thin-layer chromatography and paper chromatography. It was found that both the strains contain meso-type diaminopimelic acid (meso-DAP) as the amino acid component and galactose, glucose and ribose as sugar components. However, the presence of LL-type diaminopimelic acid (LL-DAP) which is an indispensable amino acid component of actinomycetes belonging to the genus *Streptomyces,* is not observed in *Streptomyces capreolus.* Accordingly, it is deemed that further researches will be necessary to finally determine whether or not both the strains belong to the genus *Streptomyces*. The results of the amino acid component analysis are in agreement with those shown in page 191 of "Classification and Identification of Microorganisms" compiled by Takeji Hasegawa and published by Gakkai Shuppan Center. At any rate, from the foregoing results, it is apparent that the strain MG265-CF3 is akin to *Streptomyces capreolus st.* ISP 5225. Accordingly, the strain MG265-CF3 is now identified as *Streptomyces capreolus* MG265-CF3.

Oxanosine can be prepared by aerobic culture of strain MG265-CF3 in a culture medium containing nutrients utilizable by actinomycetes. The invention also provides a process for the propagation of the microorganism *Streptomyces capreolus* MG265-CF3, which process comprises aerobically culturing cells of said microorganism in a culture medium containing a source of assimilable carbon, a source of assimilable nitrogen and, optionally, at least one inorganic salt, said culture medium being substantially free of other microorganisms.

Known nutrient sources which have heretofore been used for culturing actinomycetes can be used in the present invention. As the carbon source, there can be used, for example, glucose, glycerol, sucrose, dextrin and galactose, and mixtures thereof. As an inorganic or organic nitrogen source, there can be used, for example, ammonium chloride, ammonium sulfate, urea, ammonium nitrate, sodium nitrate, peptone, meat extract, yeast extract, dry yeast, corn steep liquor, soybean meal, cotton seed meal, Casamino acid, Bacto-Soytone, soluble vegetable protein and oat meal. These nitrogen sources may be used singly or in the form of a mixture of two or more of them. Inorganic salts such as sodium chloride, calcium carbonate, magnesium sulfate, copper sulfate, zinc sulfate, iron sulfate, manganese chloride and phosphates may be added according to need. Furthermore, organic substances such as amino acids and nucleic acids and inorganic substances, capable of promoting growth of the strain of the present invention and production of oxanosine, may be added as appropriate.

A liquid culturing method, especially submerged stirring culture, is preferred as the culturing method. It is preferred that culturing be carried out at 25 to 35°C under a neutral or weakly acidic pH condition. In the case of liquid culture, Oxanosine is ordinarily produced and accumulates in the culture broth when culture is conducted for 3 to 6 days. When the amount formed becomes largest, culture is stopped and the oxanosine is purified and isolated from the filtrate.

Methods customarily adopted for isolation of metabolites of microorganisms from culture broths may be employed for purification and isolation of Oxanosine from the culture filtrate. Oxanosine is soluble in water, methanol and dimethyl sulfoxide but insoluble or hardly soluble in ordinary organic solvents such as ethanol and acetone. Accordingly, methods customarily used for purification of nucleic acid-type antibiotic substances can be ordinarily adopted. For example, an adsorption-desorption method using active carbon powder or a porous adsorbent resin such as Amberlite XAD-2 and a column chromatography method using Avicel or Sephadex LH-20 may be appropriately combined and used for purification and isolation.

More specifically in one embodiment, the pH value of the culture filtrate is adjusted to 6.5 and the Oxanosine adsorbed on active carbon powder. The active carbon powder is washed with water and elution is effected with 50% aqueous acetone. The active fraction is concentrated and freeze-dried. The brown crude powder obtained is dissolved in methanol and insoluble substances are removed. The soluble substance is dried and dissolved in water, and the solution is subjected to adsorption treatment with an active carbon powder column and elution is carried out according to the linear concentration gradient elution method using water and 50% aqueous acetone. The active fraction is concentrated and freeze-dried. The obtained light brown crude powder containing Oxanosine is dissolved in a small amount of water and charged on a column of Avicel, and development is carried out with aqueous alcohol and elution is conducted while increasing the water content stepwise. The active fraction is concentrated and freeze-dried. The refined powder is treated with water or aqueous alcohol to obtain needle crystals of Oxanosine tinted lightly gray.

The physicochemical properties of the Oxanosine thus obtained will now be described.

1. Appearance:
   Grayish white needle crystals.

2. Elementary analysis:
   C=42.55%, H=4.41%, N=19.84%, O=33.54%.

3. Molecular formula (molecular weight):
   $C_{10}H_{12}N_4O_6$ (284.23).

4. Melting point:
Oxanosine has no definite melting point or decomposition point, but it is gradually decomposed at temperatures higher than 199°C.

5. Specific rotation power:
$[\alpha]_D^{23}=-36.7$ (C=0.3, $H_2O$).

6. Ultraviolet absorption spectrum:
The ultraviolet absorption spectrum is shown in Figure 1 of the accompanying drawings. Ultraviolet absorption and molecular extinction coefficient values in water, 0.1N hydrochloric acid and 0.1N sodium hydroxide are as follows:
$\lambda_{max}^{H_2O}$ (logε): 247 (4.08), 288 (3.93)
$\lambda_{max}^{0.1N-HCl}$(log ε): 249 (4.05), 288 (3.91)
$\lambda_{max}^{0.1N-NaOH}$ (log ε): 272 (3.96)

7. Infrared absorption spectrum:
The infrared absorption spectrum determined by using potassium bromide tablets is shown in Figure 2 of the accompanying drawings. The absorption peak values (wave number, $cm^{-1}$) are as follows:
3380, 3100—3000, 2770, 1795, 1770, 1655, 1640, 1585, 1550, 1455, 1410, 1390, 1350, 1325, 1310, 1270—1260, 1240, 1225, 1205, 1195, 1125, 1110, 1085, 1070, 1055, 1030, 1025, 1015, 1005, 990, 950, 940, 905, 870, 855, 845, 820, 795, 760, 750, 730, 675.

8. Solubility in solvents:
Soluble in water, methanol and dimethyl sulfoxide but insoluble or hardly soluble in organic solvents such as ethanol, acetone, ethyl acetate, ether and benzene.

9. Color reactions:
Positive to Rydon-Smith reaction and 5% sulfuric acid reaction but negative to ninhydrin reaction, tetrazolium chloride reaction and Sakaguchi reaction.

10. Rf values in thin-layer chromatography:
A silica gel thin layer (Kieselgel 60 $F_{254}$, 0.25 mm Merck) is used, and development is carried out with n-butanol/acetic acid/water (12/3/5 volume ratio) and n-propanol/pyridine/acetic acid/water (15/10/3/12 volume ratio) and the obtained Rf values are 0.52 and 0.86, respectively.

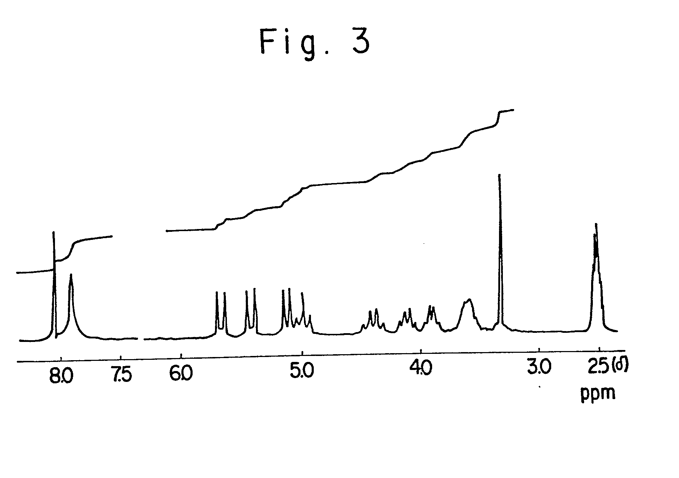

11. $^1$H-Nuclear magnetic resonance spectrum:
The $^1$H-nuclear magnetic resonance spectrum determined in deuterated dimethyl sulfoxide by using tetramethylsilane as the internal standard and 100 MHz apparatus is shown in Figure 3 of the accompanying drawings.

12. $^{13}$C-Nuclear magnetic resonance spectrum:
In the $^{13}$C-nuclear magnetic resonance spectrum determined in deuterated dimethyl sulfoxide by using tetramethylsilane as the internal standard, the chemical shift values (δ values) are as follows:
61.1, 70.3, 73.8, 85.3, 86.5, 110.9, 136.61, 153.1, 153.9, 159.8.

From the results of X-ray crystal analysis, it was found that Oxanosine has the following structural formula (I):

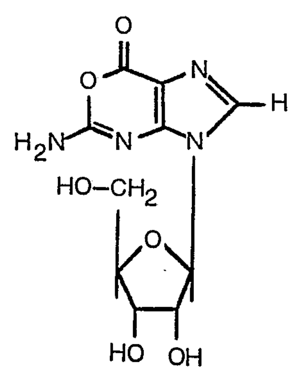

(I)

The biological activities of Xanosine will now be described.

1. Antimicrobial spectrum:
The antimicrobial spectrum of Oxanosine determined in 0.5% peptone agar is shown in Table 2 below. As is seen from Table 2, Oxanosine has a relatively strong growth inhibiting action to gram-negative

bacteria such as *Escherichia coli* K-12, *Escherichia coli* ML 1629 (multiresistant strain), bacteria belonging to the genus *Proteus* and bacteria belonging to the genus *Shigaella* but it does not show growth inhibiting action to gram-positive bacteria.

TABLE 2

| Test organism | Minimum growth inhibition concentration (mcg/ml) |
|---|---|
| *Staphylococcus aureus* FDA 209P | >100 |
| *Staphylococcus aureus,* Smith strain | >100 |
| *Micrococcus flavus* FDA 16 | >100 |
| *Bacillus subtilis* PCI 219 | >100 |
| *Bacillus subtilis* NRRLB-558 | >100 |
| *Sarcina lutea* PCI 1001 | >100 |
| *Corynebacterium bovis* 1810 | >100 |
| *Escherichia coli* NIHJ | >100 |
| *Escherichia coli* K-12 | 12.5 |
| *Escherichia coli* ML1629 (multi-resistant strain) | 25 |
| *Shigella dysenteriae* JS 11910 | 25 |
| *Shigella flexneri* 4B JS 11811 | 6.25 |
| *Shigella sonnei* JS 11746 | 25 |
| *Salmonella typhi* T-63 | >100 |
| *Salmonella enteritidis* 1891 | >100 |
| *Proteus vulgaris* OX-19 | 25 |
| *Proteus mirabilis* IFM OM-9 | 12.5 |
| *Proteus rettgeri* GN 311 | >100 |
| *Proteus rettgeri* GN 466 | 12.5 |
| *Serratia marcescens* | >100 |
| *Pseudomonas aeruginosa* A3 | >100 |
| *Klebsiella pneumoniae* PCI 602 | >100 |
| *Candida albicans* 3147 | >100 |
| *Mycobacterium smegmatis* 607 | >100 |

2. Anti-HeLa activity:
The results of examination of the anti-HeLa activity of Oxanosine are shown in Table 3.

TABLE 3

| Concentration (mcg/ml) | Cells ($\times 10^5$/ plate)* | Percent inhibition (%) | $IC_{50}$ (mcg/ml) |
|---|---|---|---|
| 100 | 1.08 | 100 | |
| 10 | 8.68 | 31.25 | ca. 30.92 |
| 1 | 10.31 | 15.33 | |
| 0.1 | 11.70 | 1.76 | |
| 0 | 11.88 | | |

Note: * number of cells after 72 hours' culturing by addition of Oxanosine.

As is apparent from Table 3, the anti-HeLa activity value ($IC_{50}$) of Oxanosine is about 30.92 mcg/ml.

3. Effect on mouse leukemia L-1210 cells:

Cells ($1\times 10^5$) of leukemia L-1210 were intraperitoneally implanted in each 8-week-old female mouse of the CDF series (5 mice per group). A solution of Oxanosine in a physiological saline solution was administered intraperitoneally once a day continuously for 9 days. The carcinostatic effect was evaluated based on the life-prolonging effect (T/C, %). The obtained results are shown in Table 4. It is seen that Oxanosine has a life-prolonging effect on tumor-bearing mice (leukemia L-1210). T/C, %=100×(days of survival of the group to which Oxanosine was administered)/(days of survival of control group to which Oxanosine was not administered).

TABLE 4

| Dose (μg/mouse/day) | Life-prolonging effect (T/C, %) |
|---|---|
| 1000 | 160 |
| 500 | 153 |
| 250 | 120 |
| 125 | 120 |
| 62.5 | 100 |
| 0 | 100 |

4. Acute toxicity to mice:

The acute toxicity value ($LD_{50}$) of Oxanosine to mice is larger than 200 mg/Kg (iv). The acute toxicity of Oxanosine is thus lower than those of various known nucleic acid-type antibiotic substances.

It can therefore be seen that Oxanosine may be expected to be both chemotherapeutic agent and also a carcinostatic agent.

The following Example illustrates the present invention. Throughout the description percentages are by weight unless the context requires otherwise.

Example

A 500 ml-capacity Sakaguchi flask for a reciprocating type shaking machine was charged with 125 ml of a culture medium (pH value=7.0) comprising 1% of glucose, 1% of glycerol, 1% of sucrose, 0.5% of oat meal, 2% of soybean meal (Prorich supplied by Ajinomoto Co.), 1% of pressed yeast (supplied by Oriental Kobo Kogyo Co.), 0.5% of casamino acid (supplied by Difco Co.) and 0.1% of calcium carbonate. The culture medium was sterilized at 120°C for 20 minutes in an autoclave. The culture medium was inoculated with one platinum loop of MG265-CF3 (Fermentation Research Institute Deposition Receipt No. 5735). Culture was carried out for 2 days at 28°C under shaking at 120 rpm.

Separately, a 500 ml-capacity, baffle-provided Erlenmeyer flask for a rotary type shaking machine was charged with 120 ml of a culture medium (pH value=7.4) comprising 2% of galactose, 2% of dextrin, 1% of Bacto-Soytone (supplied by Difco Co.), 0.5% of corn steep liquor (supplied by Ajinomoto Co.), 0.2% of ammonium sulfate, 0.2% of calcium carbonate and 0.05% of a 1:1 mixture of silicone KM-70 (supplied by Shinetsu Kagaku Kogyo Co.) and soybean oil (Japanese Pharmacopoeia). The culture medium was sterilized at 120°C for 20 minutes in an autoclave. Then, 12 ml of the first-mentioned culture medium was added to the charge of the flask and culturing was conducted at 28°C for 5 days under shaking at 180 rpm.

The resulting culture broth was filtered at a pH value of 6.5 to obtain 11 l of the filtrate. (The titer was determined according to the cup method using *Escherichia coli* K-12 as a test organism on peptone-agar plate, and the titer of the culture filtrate was designated as 1 unit (U)/ml). The filtrate was passed through a column packed with 500 ml of active carbon powder for chromatography (supplied by Wako Junyaku Co.) for the Oxanosine to adsorb on the active carbon. The column was washed with water and elution was carried out according to the acetone linear concentration gradient elution method using 1500 ml of water and 1500 ml of 50% aqueous acetone. Active fractions were collected, concentrated under reduced pressure and freeze-dried to obtain 3.56 g of dark brown crude powder (3 U/mg).

This powder was treated with 85 ml of methanol, and the soluble portion was dried under reduced pressure to obtain 1.29 g of brown crude powder (7 U/mg). The powder was dissolved in 43 ml of water and Oxanosine was adsorbed on a column packed with 180 ml of active carbon powder. The column was washed with water and elution was carried out according to the acetone linear concentration gradient elution method using 400 ml of water and 400 ml of 50% aqueous acetone. Active fractions were collected, concentrated under reduced pressure and freeze-dried to obtain 294 mg of pale brown powder 28 U/mg).

The powder was then dissolved in 11 ml of water, and the solution was charged on a column packed with 600 ml of Avicel equilibrated with ethanol/water (95/5). The elution was carried out with 1200 ml of ethanol/water(95/5) and then with 1200 ml of ethanol/water (90/10). Active fractions were collected, concentrated under reduced pressure and freeze-dried to obtain 82.8 mg of pale brownish powder (95 U/mg). The powder was dissolved in 6.4 ml of warm water and the solution was naturally cooled overnight to obtain 64.9 mg of Oxanosine in the form of slightly grayish needle crystals (117 U/mg).

**Claims**

1. A compound represented by the following formula (I):

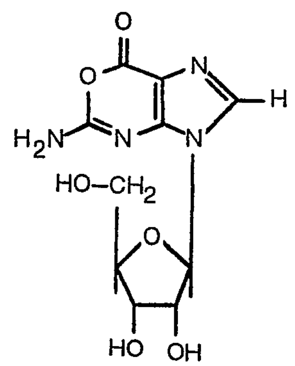

(I)

and acid addition salts thereof.

2. A process for the preparation of a compound of formula (I) as defined in claim 1 or an acid addition salt thereof, which process comprises culturing *Streptomyces capreolus* MG265-CF3 (ATCC 31963), collecting said compound from the culture broth and, if desired, converting said compound to an acid addition salt thereof.

3. A pharmaceutical composition which comprises a compound of formula (I) as defined in claim 1 or an acid addition salt thereof as active ingredient, together with a pharmaceutically acceptable carrier or diluent.

4. *Streptomyces capreolus* MG265-CF3 (ATCC 31963).

5. A process for the propagation of the microorganism *Streptomyces capreolus* MG265-CF3, which process comprises aerobically culturing cells of said microorganism in a culture medium containing a source of assimilable carbon, a source of assimilable nitrogen and, optionally, at least one inorganic salt, said culture medium being substantially free of other microorganisms.

**Patentansprüche**

1. Verbindung der folgenden Formel (I):

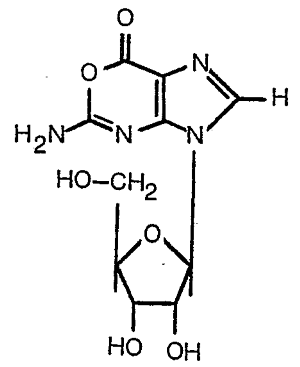

(I)

und deren Säureadditionssalze.

2. Verfahren zur Herstellung einer Verbindung der Formel (I), wie sie in Anspruch 1 definiert ist, oder eines Säureadditionssalzes davon, dadurch gekennzeichnet, daß man Streptomyces capreolus MG265-CF3 (ATCC 31963) züchtet, die Verbindung aus der Kulturbrühe gewinnt und gewünschtenfalls die Verbindung in ihr Säureadditionssalz umwandelt.

3. Pharmazeutische Zubereitung enthaltend eine Verbindung der Formel (I) gemäß Anspruch 1 oder ein Säureadditionssalz davon als Wirkstoff in Kombination mit einem pharmazeutisch annehmbaren Träger oder Verdünnungsmittel.

4. Streptomyces capreolus MG265-CF3 (ATCC 31963).

5. Verfahren zur Vermehrung des Mikroorganismus Streptomyces capreolus MG265-CF3, dadurch gekennzeichnet, daß man zellen des Mikroorganismus aerob in einem Kulturmedium züchtet, welches eine Quelle für assimilierbaren Kohlenstoff, eine Quelle für assimilierbaren Stickstoff und gegebenenfalls mindestens ein anorganisches Salz enthält und welches Kulturmedium im wesentlichen frei von anderen Mikroorganismen ist.

**Revendications**

1. Un composé représenté par la formule suivante (I):

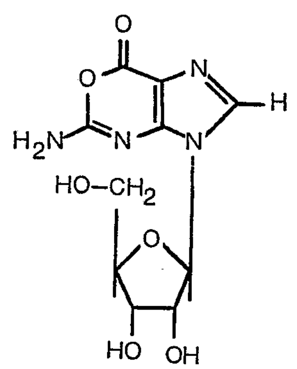

(I)

et ses sels d'additions d'acides.

2. Un procédé de préparation d'un composé de formule (I) tel que défini dans la revendication 1 ou d'un sel d'addition d'acide en dérivant, ce procédé consistant à cultiver le Streptomyces capreolus MG265-CF3 (ATCC 31963), à recueillir ledit composé du bouillon de culture et, le cas échéant, à convertir ce composé en un sel d'addition d'acide en dérivant.

3. Une composition pharmaceutique qui comprend un composé de formule (I) tel que défini dans la revendication ou un sel d'addition d'acide en dérivant agissant comme ingrédient actif anisi qu'un véhicule ou diluant pharmaceutiquement acceptable.

4. Streptomyces capreolus MG265-CF3 (ATCC 31963).

5. Un procédé de multiplication du micro-organisme Streptomyces capreolus MG265-CF3, ce procédé consistant à cultiver par voie aérobie des cellules de ce micro-organisme dans un milieu de culture contenant une source de carbone assimilable, une source d'azote assimilable et éventuellement, au moins un sel minéral, ce milieu de culture étant sensiblement exempt d'autres micro-organismes.

Fig. 1

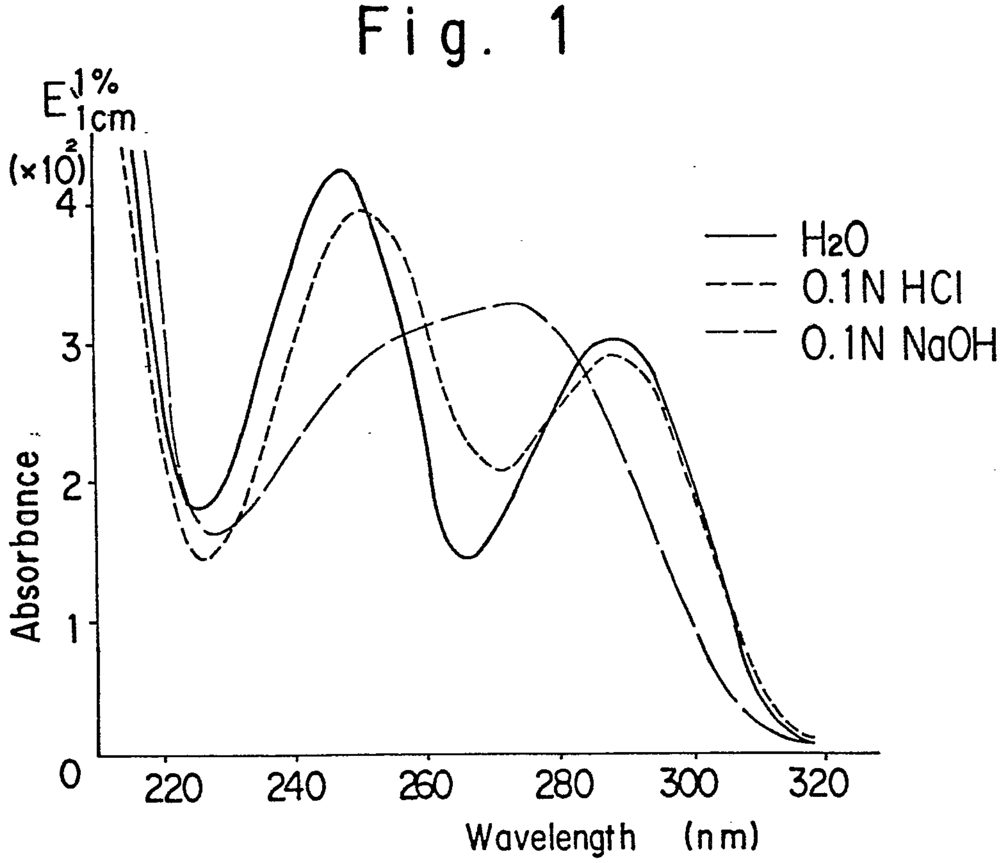

$E^{1\%}_{1cm}$
(×10²)
Absorbance
0
1
2
3
4
220
240
260
280
300
320
Wavelength (nm)
$H_2O$
0.1N HCl
0.1N NaOH


Fig. 2

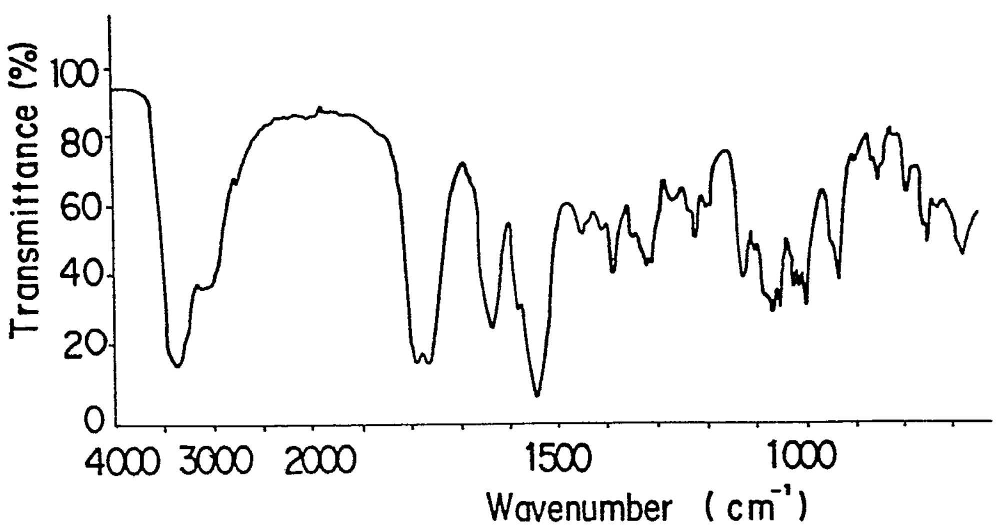

Transmittance (%)
0
20
40
60
80
100
4000
3000
2000
1500
1000
Wavenumber (cm⁻¹)

Fig. 3